## Europäisches Patentamt
## European Patent Office
## Office européen des brevets

(19)

(11) Veröffentlichungsnummer: **0 071 211**
**B1**

(12) # EUROPÄISCHE PATENTSCHRIFT

(45) Veröffentlichungstag der Patentschrift:
**07.01.87**

(51) Int. Cl.⁴: **D 04 H 13/00,** A 61 F 13/00,
A 61 L 15/00, A 61 H 33/04

(21) Anmeldenummer: **82106680.0**

(22) Anmeldetag: **23.07.82**

(54) **Flexibler, flächiger, Sand enthaltender Schichtkörper zur äusserlichen Anwendung bei der Körperbehandlung.**

(30) Priorität: **27.07.81 DE 3129526**

(43) Veröffentlichungstag der Anmeldung:
**09.02.83 Patentblatt 83/6**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

(56) Entgegenhaltungen:
**DE - A - 2 742 030**
**DE - A - 2 855 059**
**FR - A - 2 022 947**
**NL - A - 7 212 202**
**US - A - 4 250 172**

(73) Patentinhaber: **Tesch, Günter Horst, Avenue Jean-Marie-Musy 15, CH-1700 Fribourg (CH)**

(72) Erfinder: **Tesch, Günter Horst, Avenue Jean-Marie-Musy 15, CH-1700 Fribourg (CH)**

(74) Vertreter: **Lesser, Karl-Bolko, Dipl.-Ing., European Patent Attorney Johanneskirchnerstrasse 149a, D-8000 München 81 (DE)**

# Beschreibung

Die Erfindung betrifft einen flexiblen, flächigen, Sand enthaltenden Schichtkörper gemäss Oberbegriff des Anspruches 1.

Unter Körperbehandlung im Sinne der Erfindung sei die Behandlung sowohl des menschlichen, als auch des tierischen Körpers verstanden, wobei natürlich zweckentsprechende Ausführungsformen der Erfindung zur Anwendung kommen. Die Behandlung kann Heilzwecken dienen oder auch für die Kosmetik oder zur Entspannung bestimmt sein.

In der Heilkunde ist das Auflegen von Kompressen, Wickeln oder Umschlägen auf die zu behandelnden Körperteile sehr geläufig. Diese Auflagen bestehen etwa aus mehreren Lagen von Leinen oder Mull. Sie werden je nach Erfordernis mit heissem oder kaltem Wasser getränkt, um die Körperstelle zu erwärmen oder um ihr im Gegenteil Wärme zu entziehen. In vielen Fällen werden auch Wirkstoffe wie Leinsamen, Kartoffelschnitzel, Heublumen und dergleichen in die Wickel gepackt. Derartige Wickel haben ein geringes Wärmehaltungsvermögen, was bewirkt, dass der thermische Effekt sehr rasch abklingt. Der Wickel muss oft erneuert werden.

Es ist in der DE-A-2 742 030 vorgeschlagen worden, im Rahmen einer Behandlung mit konstanter, fallender oder ansteigender Temperatur, trockene oder feuchte Wärme abgebende medizinische Allzweckpackungen zu verwenden, die ein unterteiltes, aus Textilien bestehendes, zum Teil mit feinem Sand als Träger für therapeutische Stoffe gefülltes, siebartiges Kammersystem aufweisen. Die Packungen können ohne und mit in Wasser gelösten therapeutischen Stoffen versetzt angewandt werden. Die Packungen bestehen aus Stoffsäcken, die im Abstand von etwa 6 cm mittels Quernähten in mehrere Kammern unterteilt sind. Damit die Quernähte keine Hohlräume zwischen den Kammern und der Haut erzeugen, ist vorgesehen, die Kammern nur lose mit Sand zu füllen. Durch diese Massnahme soll die Packung gleichmässig auf der Haut aufliegen. Dies ist jedoch nur zum Teil der Fall, indem durch die lose Füllung der Kammern Verlagerungen des Sandes auftreten, wodurch die Wärmeeinwirkung auf die Haut ungleichmässig wird. Bei einer Verletzung der Textilwandung rieselt ausserdem der Sand heraus. Eine rationelle Fliessbandfertigung ist nicht möglich, da der Sand nach der Fertigung der Kammern eingefüllt werden muss und die Kammern erst dann verschlossen werden können.

Aufgabe der Erfindung ist es, einen flexiblen, flächigen Schichtkörper zur äusserlichen Anwendung bei der Körperbehandlung zu schaffen, der gegenüber dem vorbeschriebenen Stand der Technik einige Vorteile besitzt, die ihn universeller verwendbar machen und die eine rationellere Fertigung erlauben.

Durch die EP-Anmeldung 82 106 681 8 (Veröffentlichungsnummer 0 071 212) soll ein flexibler, flächiger, Sand enthaltender Schichtkörper, bei dem eine aktiv nadelfähige Faserschicht und eine Gegenschicht durch eine Gesteinspartikel, wie Sand, Splitt oder dergleichen enthaltende Zwischenschicht hindurch miteinander vernadelt sind, unter Schutz gestellt werden.

Es wurde nun festgestellt, dass, um diese Aufgabe zu lösen, ein solcher Schichtkörper auch zur äusserlichen Behandlung bei der Körperbehandlung geeignet ist.

Das Vernadeln von Gesteinspartikeln zwischen einer Faserschicht und einer Gesteinsschicht bietet verschiedene Vorteile. Erstens erlaubt es eine rationelle Fertigung auf dem Fliessband, indem die einzelnen Schichten kontinuierlich auf ein Förderband abgelegt, vernadelt und zu einem Wickel aufgewickelt oder zu Platten geschnitten werden können. Sodann lässt sich der Schichtkörper auf eine beliebige Flächenform zerschneiden, wobei die Sandpartikel an den Schnitträndern durch die hindurchgenadelten Haltefasern am Herausrieseln gehindert werden. Es können dann auch Öffnungen herausgeschnitten werden, z.B. für Gesichtsmasken. Bei Verwendung von Sand mit stark variierender Korngrösse erfolgt keine Entmischung nach Korngrösse, da die zahlreich über die ganze Fläche verteilt durchgezogenen Haltefasern die Sandkörner an einer seitlichen Verschiebung hindern.

Durch Nadelung hergestellte Schichtkörper mit eingelagerten körnigen Stoffpartikeln sind durch die CH-A-496 462 und die DE-A-2 855 059 bekannt geworden. Bei den eingelagerten Stoffpartikeln handelt es sich jedoch um Wirkstoffe, wie Aktivkohle, Düngstoffe etc., die eine relativ geringe Dichte haben und die auch nicht das Wärmespeichervermögen von Gesteinspartikeln aufweisen. Diese bieten ausserdem den Vorteil, dass sie dem Schichtkörper ein relativ grosses Flächengewicht erleihen, wodurch er einen gleichmässigen Druck auf die Körperhaut ausübt. Im Gegensatz zu den im Stand der Technik aufgeführten Wirkstoffen, wie Aktivkohle, locken, Fasern, die von ihrer Natur aus dem Durchstich der Filznadel keinen Widerstand entgegensetzen, da sie entweder leicht zerbröckeln oder leicht durchstochen werden, war es gegenüber dem Einsatz von Gesteinspartikeln überraschend, dass Filznadeln durch eine Schicht von Gesteinspartikeln hindurchstechbar sind. Es bestand ein Vorurteil, dass die Nadeln von den Sandkörnern zerstört werden. Es hat sich jedoch gezeigt, dass dies nicht der Fall ist, offenbar weil die Sandkörner auf der elastischen Faserauflage ausweichen. Überraschend war auch, dass die Sandkörner beim fertigen Erzeugnis nicht zwischen den Fasern der aktiv nadelfähigen Faserschicht hindurchrieseln. Durch die Nadelung wird nämlich eine solche Verdichtung der Faserschicht erzielt, dass kein Sand mehr herausrieselt.

Bei Verwendung eines solchen Schichtkörpers können in der Gesteinspartikelschicht Wirkstoffe in Körner- oder Pulverform verteilt vorliegen. Da die Wirkstoffe, wie z.B. Heublumen, Kamillenblüten, Mandelkeime, sehr leicht sind, tritt eine Entmischung bei loser Anhäufung in Kammern sehr leicht ein, was bei dem erfindungsgemäss verwendeten Schichtkörper nicht der Fall ist. Gleichzeitig wird bei diesem Schichtkörper noch der Vorteil erzielt, dass die Wirkstoffe gleichmässig verteilt verbleiben, auch wenn der Schichtkörper verformt, geschüttelt oder auf die Kante gestellt wird.

Ausführungsbeispiele der Erfindung werden anhand der Zeichnung, in der Teilstücke der Schichtkörper schematisch dargestellt sind, näher erläutert.

Es zeigen:

Figur 1 einen Schichtkörper im Schnitt,

Figur 2 eine andere Ausführungsform des Schichtkörpers im Schnitt,

Figur 3 eine weitere Ausführungsform im Schnitt in einem Zwischenstadium der Herstellung des Schichtkörpers,

Figur 4 denselben Schichtkörper im Schnitt nach Fertigstellung,

Figur 5 eine weitere Ausführungsform im Schnitt,

Figur 6 denselben Schichtkörper in der Draufsicht.

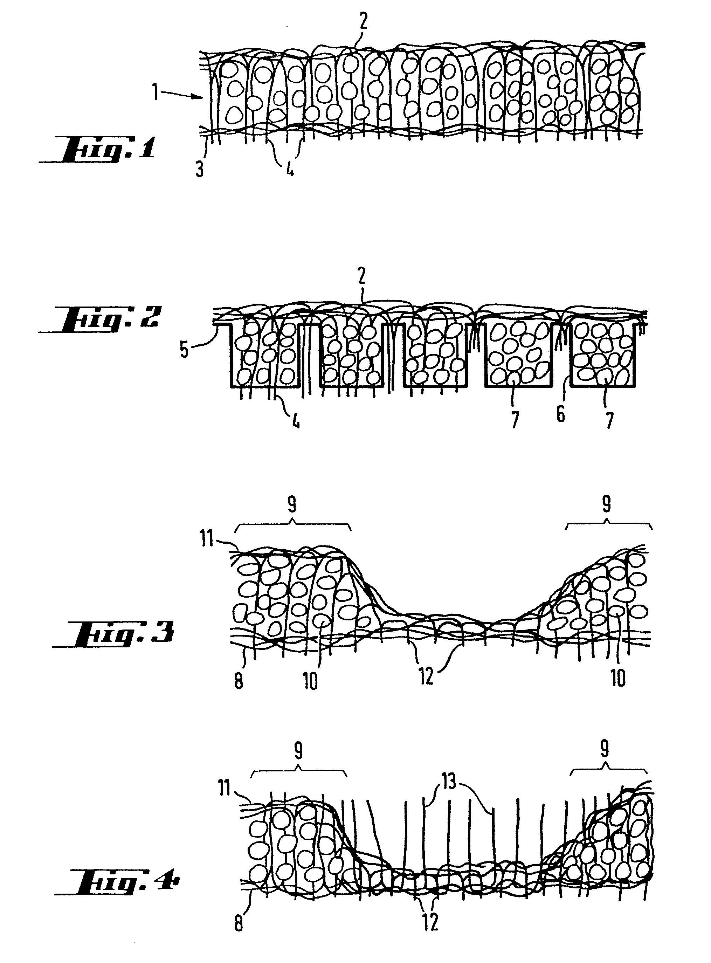

In der Ausführungsform des Schichtkörpers gemäss Figur 1 ist eine Schicht 1 aus Gesteinspartikeln zwischen einer aktiv nadelfähigen Faserschicht 2 und einer Gegenschicht 3 eingeschlossen. Die Schichten 2 und 3 sind durch die Zwischenschicht 1 hindurch miteinander vernadelt. Die Vernadelung kann nach einem in der Nadelfilztechnologie bekannten Nadelverfahren erfolgen, wie es z.B. von R. Krcma im «Handbuch der Textilverbundstoffe», Deutscher Fachverlag, Frankfurt a/Main, 1970, S. 198 bis 202, beschrieben ist. In dieser Technologie werden zum Vernadeln am häufigsten Filzandeln mit dreieckigem Nadelschaft und seitlichen, gegen die Spitze zu gerichteten Widerhaken verwendet.

Gebräuchlich sind auch andere Formen, wie Gabelnadeln und Loop-Nadeln. Auch die im Buch von R. Krcma erwähnten Nähwirknadeln lassen sich für die Vernadelung des Schichtkörpers entsprechend verwenden. Die Filznadeln erfassen beim Einstechen in die Faserschicht 2 einzelne oder Büschel von Fasern aus der Faserschicht und verflechten sie mit der Gegenschicht 3. Die Faserschicht 2 muss zu diesem Zweck aktiv nadelfähig sein, d.h. es sollen sich Fasern aus der Schicht erfassen lassen, wobei ein Teilstück dieser Fasern noch in der Schicht verankert bleibt.

Durch den Nadelvorgang werden nicht nur die beiden Schichten 2 und 3 miteinander verbunden. Es werden auch die Gesteinspartikel der Zwischenschicht 1 durch die zahlreich auf die ganze Fläche des Schichtkörpers verteilt durchgezogenen Haltefasern 4 am seitlichen Verschieben gehindert. Dadurch ist es möglich, den Schichtkörper in beliebige Flächenformen zu zerschneiden, ohne dass die Gesteinspartikel in wesentlicher Menge aus der Schnittkante herausrieseln.

Der bahnförmig hergestellte Schichtkörper kann aber auch durch Trennschweissen in einzelne, handelsübliche Grössen zerteilt werden. Wenn thermoplastische Fasern verwendet werden, lassen sich insbesondere dabei auch die Kanten der Einzelstücke durch Schweissen verfestigen. Gegebenenfalls wird im Bereich der Kante U-förmig ein Streifen aus thermoplastischem Material unter Druck- und Hitzeeinwirkung angeschweisst, wodurch der Schichtkörper ein gefälligeres Aussehen erhält.

Die Schicht 1 von Gesteinspartikeln kann aus natürlichem Gesteinsmaterial von geringer Korngrösse, z.B. Sand, bestehen, der definitionsgemäss eine Korngrösse von 0,02 bis 2 mm aufweist. Aber auch Grobsand und sogar Kies, und feinkörniger Splitt sind verwendbar, soweit sie das Durchstossen der Filznadeln nicht ganz verhindern. Vorteilhafte gemeinsame Merkmale dieser Materialien sind ihr relativ hohes Wärmehaltungsvermögen, ihr relativ grosses Gewicht bezogen auf eine bestimmte Schichtdicke und ihr inertes Verhalten gegenüber den Benetzungsflüssigkeiten bei der Verwendung und gegenüber gegebenenfalls beigemischten, der Körperbehandlung dienenden Wirkstoffen.

Lehm als sandhaltiger Ton oder Talcum sind ebenfalls geeignet zum Aufbau der Schicht 1.

Die Gegenschicht 3 kann aus verschiedenartigem Material bestehen. Sie soll beim Durchstechen der Nadeln nicht aufsplittern und soll die durchgestochenen Haltefasern 4, z.B. elastisch, festhalten, z.B. durch Klemmung oder Verflechtung, d.h. die Gegenschicht 3 soll passiv nadelfähig sein. Es eignen sich hierfür z.B. Kunststofffolien aus weichelelastischem Material, Faserschichten in genügender Dichte, die durch den Nadelprozess selber noch weiter verdichtet und verfilzt werden, sodass sie die Gesteinspartikel zurückhalten, sowie adhästiv gebundene Faserverbundstoffe. Die Gegenschicht 3 kann auch aktiv nadelfähig sein, was erlaubt, den Schichtkörper zusätzlich von der Gegenseite her zu vernadeln. Es ist auch möglich, auf eine Kunststofffolie oder dergleichen als Gegenschicht 3 eine weitere aktiv nadelfähige Faserschicht aufzulegen und den Schichtkörper von beiden Seiten her zu vernadeln.

Die Faserschicht, sei es als Schicht 2 oder als Gegenschicht 3 eingesetzt, kann durch separate Nadelung vorverdichtet sein, oder sie kann auf eine Trägerschicht, wie Kunststofffolie, Faserverbundstoff oder dergleichen, aufgenadelt werden, um die Handhabung bei der Herstellung zu erleichtern, oder um das Durchsickern von feinen Gesteinspartikeln vor dem Vernadeln des Schichtkörpers zu verhindern. Als Fasermaterial kommen die verschiedensten textilen Fasern in Frage. Es können thermoplastische synthetische Fasern, wie Polypropylen oder Polyesterfasern, sein, die sich thermoplastisch verformen, verschweissen oder zertrennen lassen. Für besondere Anwendungen, z.B. für die Aussenschicht, die direkt mit der Körperhaut in Berührung gebracht wird, können saugfähige Fasern, wie Baumwolle, Zellwolle oder Viscose, eingesetzt werden. Andererseits können auch wasserquellbare oder wasserlösliche Fasern, wie Polyvinylalkoholfasern und dergleichen, verwendet werden, um nassverformbare Schichtkörper zu verfertigen. Solche eignen sich z.B. für Gesichtsmasken, die aus einem flachen Rohling ausgeschnitten und nach Benetzung der Gesichtform angepasst werden.

Als Gegenschicht kann, wie Figur 2 zeigt, auch eine Kunststoffolie 5 oder ein Faserverbundstoff verwendet werden, die mit näpfchenartigen Vertiefungen oder Noppen versehen ist, die z.B. durch Tiefziehen im warmplastischen Zustand erzielt werden. Die Vertiefungen 6 sind mit Gesteinspartikeln 7 gefüllt. In diesem Beispiel ist somit die Schicht von Gesteinspartikeln nicht zusammenhängend, sondern in zahlreiche Portionen aufgeteilt. Die Nadeleinstiche können gleichmässig dicht über die ganze Fläche des Schichtkörpers verteilt sein, sie können aber auch neben den Vertiefungen 6 vorbeigeführt werden. Im ersten Falle werden die Vertiefungen 6 siebartig durchlöchert, wobei die durchgezogenen Haltefasern 4 z.B. bei Benetzung durch Körperflüssigkeit, wie Schweiss, Blutserum, die Flüssigkeit durch Kapillarität durch die Gegenschicht hindurchleiten. Im zweiten Falle bleiben die Vertiefungen 6 unversehrt. Die Vertiefungen 6 lassen sich auch wahlweise mit verschiedenartigen Stoffpartikeln füllen, z.B. abwechselnd mehrere Reihen nebeneinander mit Sand und eine Reihe mit der Körperbehandlung dienenden Wirkstoffen, z.B. Heublumen, Leinsamen, Mandelkleie.

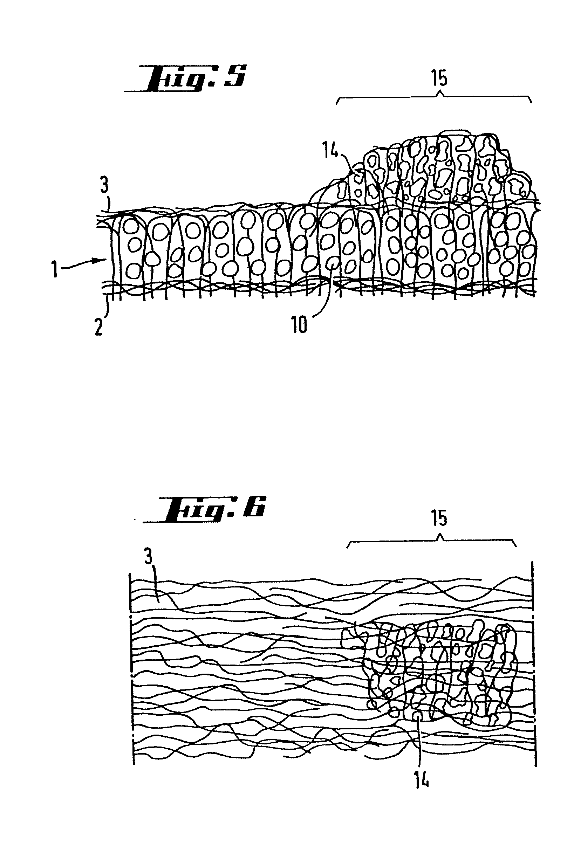

In der in Figur 3 und Figur 4 dargestellen Ausführungsform des Schichtkörpers sind auf eine aktiv nadelfähige Faserschicht 8 Reihen oder Streifen 9 von Gesteinspartikeln 10 abgelegt. Diese bilden wiederum eine unterbrochene Zwischenschicht, durch welche hindurch genadelt wird. Auf die Reihen von Gesteinspartikeln 10 wird eine aktiv nadelfähige Faserschicht 11 abgelegt und der Schichtkörper von oben streifenweise zwischen den Reihen 9 hindurch genadelt. (Haltefasern 12 in Figur 3). Hierauf wird der Schichtkörper von der Seite der Gegenschicht 8 her nochmals genadelt, jedoch mit einer gleichmässig dichten Verteilung der Einstiche (Haltefasern 13 in Figur 4). Dadurch sind die beiden Deckschichten 8 und 11 im Bereich zwischen den Reihen 9 dichter miteinander vernadelt, als durch die Reihen 9 hindurch. Durch geeignete Wahl der Dichten der Haltefasern 12 und 13 kann die Flexibilität des Schichtkörpers gesteuert werden, z.B. wird eine grössere Flexibilität in Richtung quer zu den Reihen als parallel dazu erzeugt. Die Bereiche der Reihen 9, die bei der Verwendung auf der Körperhaut aufliegen, fühlen sich weicher an, als die dichter vernadelten Bereiche zwischen den Reihen 9.

Die Ausführungsform gemäss Figur 3 und Figur 4 ist auch geeignet zum abwechslungsweisen Anordnen von Reihen 9, von denen die einen aus Sand und die anderen aus Wirkstoffpartikeln bestehen.

In Figur 5 und Figur 6 ist ein Schichtkörper dargestellt, der ähnlich wie am Beispiel gemäss Figur 1 gezeigt, aufgebaut ist. Es sind auch für dieselben Teile dieselben Bezugszeichen verwendet worden. Der Schichtkörper ist lediglich in umgekehrter Lage gezeichnet, d.h. die Faserschicht 2 liegt unten, die Gegenschicht 3 oben. Auf die letztere ist eine Schicht 14 von weiteren Partikeln, wie getrockneter Lehm, der Körperbehandlung dienenden Wirkstoffen, von Duftstoffen und dergleichen, abgelegt. Die Schicht 14 ist mittels einer weiteren, gegebenenfalls vorvernadelten Faserschicht 15 auf den Schichtkörper aufgenadelt. Wie aus der Draufsicht gemäss Figur 6 ersichtlich ist, kann der Bereich der Schicht 14 flächenmässig begrenzt sein in Anpassung an einen besonderen Verwendungszweck. Es können auch neben oder übereinander weitere solche Partikelschichten wie Schicht 14 aufgenadelt sein.

Die Wirkstoffe können ganz allgemein in Körner-, Pulver-, Flocken- oder Faserform vorliegen. Sie können, wie oben beschrieben, gesondert von den Gesteinspartikeln vorhanden sein. Sie können aber auch mit den Gesteinspartikeln vermischt sein. In diesem Falle dienen diese als Verdünnungsmittel für die Wirkstoffe bzw. zur besseren Verteilung über die ganze Fläche des Schichtkörpers.

Es können als Gesteinspartikel auch durch Aktivierung abbindbare Bindemittel verwendet werden. Als Bindemittel kann z.B. Gips verwendet werden. Damit lassen sich z.B. Verbände zur Fixierung von Knochenbrüchen herstellen. Der Schichtkörper wird unmittelbar vor dem Auflegen auf das verletzte Körperglied mit Wasser gesättigt, dem Körperglied angepasst, und das Körperglied bis zum Abbinden des Gipses ruhig gestellt. Es lassen sich mittels Bindemittelzugabe auch Schichtkörper mit einseitiger Flexibilität herstellen. Es wird als Beispiel auf die Ausführungsform gemäss Figur 3 und Figur 4 verwiesen. Die anhand dieses Beispiels oben beschriebene Anisotropie der Flexibilität des Schichtkörpers lässt sich nämlich Verstärken durch Beimischung und Aktivierung eines Bindemittels zu den Gesteinspartikeln 10, wodurch die Reihen 9 versteift werden, während die Zwischenräume flexibel bleiben.

Schliesslich können auch Gesichtsmasken, z.B. für kosmetische Anwendungen, z.B. durch Tiefziehen oder durch das Nassverformen, geformt werden. Hierbei kann eine lockere Vernadelung von Vorteil sein. Diese können mittels den Gesteinspartikeln beigemischten und nach der Formgebung aktivierten Bindemitteln versteift werden. Die Augen und die Nasenpartien können durch einfaches Ausschneiden oder Ausstanzen von Öffnungen freigelegt werden. Es ist dabei ein wesentlicher Vorteil des erfindungsgemäss verwendeten Schichtkörpers, dass die Gesteins- und gegebenenfalls Wirkstoffpartikel von den durchgenadelten Haltefasern 4 am Herausrieseln gehindert werden.

Bei Verwendung von thermoplastischen Fasern können die Ausschnitte z.B. auch mittels heissen Stanzeisen ausgeschnitten werden, wobei die Fasern an den Schnitträndern gleichzeitig miteinander verschweisst werden, sodass die Ränder nicht ausfransen können.

Die Verwendung von Sand, insbesondere von gewaschenem Quarzsand, zusammen mit sterilisierfähigem Fasermaterial, wie Baumwolle, Polypropylen, bietet den Vorteil, dass das Material hygienisch einwandfrei zubereitet werden kann und dass es sich gegenüber Körperbehandlungsflüssigkeiten völlig inert verhält. Es können Desinfektinsmittel beigegeben werden. Bei Verwendung als Gesichtsmaske können die für die Behandlung der Gesichtshaut üblichen Wirkstoffe beigegeben werden. Es kann durch den Schichtkörper hindurch massiert werden, wobei dieser den Zusammenhalt nicht verliert, wie dies bei den üblichen Fango- und dergleichen Packungen der Fall ist. Der Schichtkörper lässt sich auch als Ganzes wieder von der Haut ablösen, ohne Rückstände zurückzulassen.

Ein Schichtkörper, der in schmalen Bahnen hergestellt oder in solche zerschnitten ist, kann darüberhinaus auch als Bandage oder Wickel verwendet werden. Durch die in den Schichtkörper eingenadelten Gesteinspartikel, die dann einen kleineren Korndurchmesser aufweisen sollten, z.B. in der Grössenordnung von 0,1 bis 1,0 mm, kann eine solche Bandage oder ein solcher Wickel nicht nur Feuchtigkeit halten und gleichmässig an den Körper abgeben, es ist auch möglich, den Schichtkörper vor der Anwendung, z.B. heissem Wasser, zu erwärmen, wodurch die Bandage oder insbesondere der Wickel über einen längeren Zeitraum hinweg Wärme und Feuchte an die Körperoberfläche abgeben kann.

Gemäss einem nicht in der Zeichnung dargestellten Ausführungsbeispiel kann auch mittels dieses erfindungsgemässen Schichtkörpers auf einfache Art und Weise ein Heizkissen hergestellt werden. Ein solches besteht z.B. aus zwei flächig aufeinandergelegten Schichtkörpern gemäss der Erfindung, zwischen denen eine Folie mit eingeschweissten Widerstandsdrähten angeordnet ist. Anstelle dieser Folie kann aber auch eine herkömmliche Einlage für Heizkissen zwischen diesen beiden Schichtkörpern vorgesehen sein.

Gemäss einer bevorzugten Ausführungsform sind dabei die beiden erfindungsgemässen Schichtkörper an drei ihrer Kanten sackartig miteinander fest verbunden, während sie an ihren vierten Kanten mit Einrichtungen zum Schliessen dieses sackförmigen Gebildes, wie Druckknöpfen, einem Reissverschluss oder dergleichen versehen sind. Der Heizteil dieses Heizkissens kann dadurch zum Waschen oder Reinigen der die Umhüllung bildenden Schichtkörper herausgenommen und nach dem Säubern weider hineingeschoben werden. Durch die Anordnung der Gesteinspartikel in den beiden Schichtkörpern kann dadurch das Wärmehaltevermögen dieses Heizkissens vergrössert werden. D.h., es kann ein Heizkissen zur Verfügung gestellt werden, das auch dann noch Wärme abgibt, wenn dem Heizkissen kein Strom mehr zugeführt wird.

Je nach Grösse dieses Heizkissens kann ein solches auch als Wärmedecke ausgebildet sein.

Der Schichtkörper gemäss der Erfindung können trocken verwendet werden zur Wärmeabgabe, zur Beschwerung von Operationswunden etc., oder sie können mit wässerigen Flüssigkeiten benetzt verwendet werden. Die Benetzungsflüssigkeit kann reines Wasser oder Dispersion von Wirkstoffen in Wasser sein, z.B. Pflanzenextrakte, essigsaure Tonerde, Dispersion von Mandelöl, von Duftstoffen.

Beispiel für die Herstellung eines Schichtkörpers gemäss Figur 1:

Die Faserschicht 2 und die Gegenschicht 3 wurden identisch aus dem gleichen Material auf folgende Weise hergestellt: Auf eine Trägerfolie aus Polyäthylen von 0,1 mm Dicke wurde ein Fasergemisch von 200 g/m² von Polyester-Fasern mit einem Fasertiter von 3,3 und 17 dtex und einer Stapellänge von 90 mm abgelegt. Die Fasern wurden mit der Folie mittels konventioneller Filznadeln mit 46 Stichen/cm² vorvernadelt. Eine solche vorvernadelte Faserschicht wurde mit den Faserbärten nach oben gerichtet auf den Zufuhrtisch der Nadelmaschine gelegt, dann darauf eine Schicht von gewaschenem Quarzsand der Krongrösse 0,5 bis 0,75 mm in einer Menge von 7 kg/m² gestreut. Die Schicht wurde sodann mit einer identischen vorvernadelten Faserschicht, mit den Faserbärten nach unten gerichtet, zugedeckt. Der ganze Schichtkörper wurde mit konventionellen 25-gauge Filznadeln und mit 30 Stichen/cm² vernadelt. Es entstand ein Schichtkörper von ca. 7,4 kg/m² Flächengewicht.

Beispiel für die Herstellung eines Schichtkörpers gemäss Figur 2:

Als Gegenschicht 5 wurde eine Noppenfolie aus Polyäthylen mit zylindrischen Vertiefungen (Noppen) von 1 cm Ø und 5 mm Tiefe, 7700 Noppen/m², verwendet. Die Noppen wurden mit Quarzsand gestrichen gefüllt, dann mit einer Schicht von Polypropylenfasern, 17 dtex, Stapellänge 90 mm, 200 g/m² zugedeckt. Der Schichtkörper wurde mit konventionellen 25-gauge Filznadeln mit 30 Stichen/cm² vernadelt. Es entstand ein Schichtkörper von ca. 1,8 kg/m² Flächengewicht. Die Noppen waren durch die Nadeleinstiche perforiert worden. Der Sand konnte jedoch nicht herausfallen.

Beispiel für die Herstellung eines Schichtkörpers gemäss Figur 3 und Figur 4:

Es wurden wie im Beispiel zu Figur 1 je eine identische vorgenadelte Faserschicht 8 bzw. 11 angefertigt. Auf die Schicht 8 wurde Quarzsand, wie zuvor, in einer Menge von 5 kg/m² in parallelen Streifen nebeneinander abgelegt und dann von oben mittels konventioneller Filznadeln, jedoch in Reihenanordnung mit 30 Stichen/m² neben den Sandreihen hindurch genadelt. Der Schichtkörper wurde dann gewendet und nochmals mittels konventioneller Filznadeln, jedoch mit der üblichen gleichmässigen Verteilung der Filznadeln auf dem Nadelbrett mit 30 Stichen/cm² genadelt.

**Patentansprüche**

1. Flexibler, flächiger, Sand enthaltenden Schichtkörper, bei dem eine aktiv nadelfähige Faserschicht (2, 11) und eine Gegenschicht (3, 5, 8) durch eine Gesteinspartikel (1, 7, 10) wie Sand, Splitt oder dergleichen enthaltende Zwischenschicht hindurch miteinander vernadelt sind, dadurch gekennzeichnet, dass der Schichtkörper der äusserlichen Körperbehandlung dienende, feste Wirkstoffe (14) enthält.

2. Schichtkörper nach Ansrpuch 1, dadurch gekennzeichnet, dass die Wirkstoffe (14) in Flüssigkeiten löslich sind.

3. Schichtkörper nach Anspruch 1, dadurch gekennzeichnet, dass der Wirkstoff (14) in Flüssigkeiten lösliche Bestandteile aufweist.

4. Schichtkörper nach einem der vorhergehenden Ansprüche, wobei der Schichtkörper näpfchenartige Vertiefungen aufweist, dadurch gekennzeichnet, dass ein Teil der Vertiefungen (6) mit den Gesteinspartikeln (7) und ein anderer Teil der Vertiefungen (6) mit den festen Wirkstoffen in Pulver- oder Körnerform gefüllt sind.

5. Schichtkörper nach einem der vorhergehenden Ansprüche, dadurch gekennzeichnet, dass in der Zwischenschicht nebeneinander Bereiche mit den Gesteinspartikeln und Bereiche mit den festen Wirkstoffen in Pulver- oder Körnerform vorhanden sind.

6. Schichtkörper nach einem der vorhergehenden Ansprüche, dadurch gekennzeichnet, dass in der Gesteinspartikel (1, 7, 10) enthaltenden Zwischenschicht die festen, der Körperbehandlung dienenden Wirkstoffe verteilt sind.

7. Schichtkörper nach Anspruch 6, dadurch gekennzeichnet, dass die Wirkstoffe, bzw. solche enthaltende Stoffpartikel, als stückige, körnige oder pulverförmige Partikel vorliegen.

8. Schichtkörper nach Anspruch 6, dadurch gekennzeichnet, dass die Wirkstoffe mit den Gesteinspartikeln (1, 7, 10) adhäsiv, z.B. als Umhüllung oder als Brücke zwischen zwei Gesteinspartikeln, verbunden sind.

9. Schichtkörper nach einem der vorhergehenden Ansprüche, wobei der Schichtkörper mit einem weiteren Schichtkörper verbunden ist, dadurch gekennzeichnet, dass der weitere Schichtkörper eine Zwischenschicht mit der Körperbehandlung dienenden, festen Wirkstoffpartikeln aufweist.

10. Schichtkörper nach einem der vorhergehenden Ansprüche, dadurch gekennzeichnet, dass die Wirkstoffe als Partikelschicht (14) aussen an der Faserschicht (2) oder an der Gegenschicht (3) anliegen und dass eine weitere Faserschicht (15) durch die Wirkstoffpartikelschicht hindurch mit dem Schichtkörper vernadelt ist.

11. Schichtkörper nach einen der vorhergehenden Ansprüche, wobei der Schichtkörper verbunden ist, dadurch gekennzeichnet, dass zwischen den beiden Einzel-Schichtkörpern eine Heizfolie bzw. Heizmatte angeordnet ist.

12. Schichtkörper nach Anspruch 11, dadurch gekennzeichnet, dass die beiden Einzel-Schichtkörper, einen Sack bildend, an drei ihrer Kanten miteinander verbunden sind.

## Claims

1. Flat, flexible laminated article containing sand, in which an actively needleable fibre layer (2, 11) and a backing layer (3, 5, 8) are needled together through an intermediate layer containing mineral particles (1, 7, 10) such as sand, chippings or the like, characterised in that the laminated article contains stable active substances (14) serving for external application to the body.

2. Laminated article according to Claim 1, characterised in that the active substances are soluble in fluids.

3. Laminated article according to Claim 1, characterised in that the active substance (14) includes components soluble in fluids.

4. Laminated article according to one of the foregoing claims in which the article has pot-like recesses, characterised in that some of the recesses (6) are filled with the mineral particles (7) and others of the recesses (6) are filled with the stable active substances in powdered or granular form.

5. Laminated article according to one of the foregoing claims, characterised in that in the intermediate layer there are regions with the mineral particles alongside regions with the stable active substances in powdered or granular form.

6. Laminated article according to one of the foregoing claims, characterised in that the stable active substances serving for treatment of the body are distributed in the intermediate layer that contains the mineral particles (1, 7, 10).

7. Laminated article according to Claim 6, characterised in that the active substances or particles of material containing them are present as individual granular or powder particles.

8. Laminated article according to Claim 6, characterised in that the active substances are connected to the mineral particles (1, 7, 10) adhesively, eg. as a coating or acting as bridges between two mineral particles.

9. Laminated article according to one of the foregoing claims in which the article is connected to a further laminated article, characterised in that the further laminated article has an intermediate layer with stable particles of an active substance serving for treatment of the body.

10. Laminated article according to one of the foregoing claims, characterised in that the active substances in the form of a layer of particles (14) engage externally against the fibre layer (2) or against the backing layer (3) and that a further fibre layer (15) is needled to the laminated article through the layer of particles of acitve substances.

11. Laminated article according to one of the foregoing claims, the article being connected to a further laminated article, characterised in that a heating foil or heating mat is arranged between the two individual laminated articles.

12. Lamianted article according to claim 11, characterised in that the two individual laminated articles are connected together along three of their edges to form a bag.

## Revendications

1. Objet stratifié flexible et plat contenant du sable, dans lequel une couche de fibres (2, 11) activement aiguilletable et une couche opposée (3, 5, 8) sont aiguilletées l'une à l'autre en passant par une couche intermédiaire contenant des particules de pierraille (1, 7, 10) telles que du sable, du gravier et analogues, caractérisé en ce que l'objet stratifié contient des matières actives solides (14) servant au traitement externe du corps.

2. Objet stratifié selon la revendication 1, caractérisé en ce que les matières actives sont solubles dans les liquides.

3. Objet stratifié selon la revendication 1, caractérisé en ce que la matière active (14) comprend des constituants solubles dans les liquides.

4. Objet stratifié selon l'une quelconque des revendications 1 à 3, l'objet stratifié comprenant des cavités en forme de petits godets, caractérisé en ce qu'une partie des cavités (6) est remplie de particules de pierraille (7) et une autre partie des cavités (6) de matières actives solides sous la forme de granules ou de poudre.

5. Objet stratifié selon l'une quelconque des revendications 1 à 4, caractérisé en ce que des zones de particules de pierraille et des zones de matières actives solides en forme de granules ou de poudre sont présentes côte à côte dans la couche intermédiaire.

6. Objet stratifié selon l'une quelconque des revendications 1 à 5, caractérisé en ce que les matières actives solides servant au traitement du corps sont réparties dans la couche intermédiaire contenant des particules de pierraille (1, 7, 10).

7. Objet stratifié selon la revendication 6, caractérisé en ce que les matières actives, voire les particules de matières contenant celles-ci, se présentent sous la forme de morceaux, de granules ou de poudre.

8. Objet stratifié selon la revendication 6, caractérisé en ce que les matières actives sont reliées aux particules de pierraille (1, 7, 10) adhésivement, par exemple, par enrobement ou pontage entre deux particules de pierraille.

9. Objet stratifié selon l'une quelconque des revendications 1 à 8, l'objet stratifié étant relié à un autre objet stratifié, caractérisé en ce que l'autre objet stratifié comprend une couche intermédiaire contenant des particules de matières actives solides servant au traitement du corps.

10. Objet stratifié selon l'une quelconque des revendications 1 à 9, caractérisé en ce que les matières actives sont adjacentes, en tant que couche particulaire (14), extérieurement à la couche de fibres (2) ou à la couche opposée (3) et en ce qu'une autre couche de fibres (15) est aiguilletée avec l'objet stratifié par l'intermédiaire de la couche de particules de matières actives.

11. Objet stratifié selon l'une quelconque des revendications 1 à 10, l'objet stratifié étant relié à un autre objet stratifié, caractérisé en ce qu'une feuille chauffante ou un feutre chauffant est disposé entre les deux objets stratifiés individuels.

12. Objet stratifié selon la revendication 11, caractérisé en ce que les deux objets stratifiés individuels sont reliés l'un à l'autre par trois de leurs côtés en formant un sac.

Fig. 1

Fig. 2

Fig. 3

Fig. 4

**Fig. 5**

**Fig. 6**